# EUROPEAN PATENT APPLICATION

(11) **EP 4 487 677 A1**
(43) Date of publication of application: **08.01.2025**
(21) Application number: 23763499.3
(22) Date of filing: 01.03.2023
(51) Int. Cl.: A01G 7/00, C09K 17/32, C12N 1/14

(54) **COMPOSITION AND METHOD FOR ENHANCING INFECTIOUSNESS OF ARBUSCULAR MYCORRHIZAL FUNGI**

(30) Priority: 04.03.2022 JP 2022033616
(71) Applicant: National University Corporation Tottori University, Tottori-shi, Tottori 680-8550 (JP)
(72) Inventor: KAMINAKA Hironori, Tottori-shi, Tottori 680-8550 (JP); TOMINAGA Takaya, Tottori-shi, Tottori 680-8550 (JP); UENO Kotomi, Tottori-shi, Tottori 680-8550 (JP); SAITO Hikaru, Tottori-shi, Tottori 680-8550 (JP)
(74) Representative: Grünecker Patent- und Rechtsanwälte PartG mbB
(86) International application number: PCT/JP2023/007612
(87) International publication number: WO 2023/167242

(57) **Abstract**

A composition and method that can act on an arbuscular mycorrhizal fungus to promote colonization of the arbuscular mycorrhizal fungus in a plant and to facilitate its symbiosis with the plant are provided. Provided is a composition for promoting colonization of an arbuscular mycorrhizal fungus, the composition comprising as an active component a secoiridoid glycoside, iridoid glycoside, or a glycoside in which a 3,4-dihydropyran ring skeleton forms at position 2 an O-glycosidic bond to a glucopyranose, or dried remains or an extract of a Gentianaceae plant. A method for improving soil by adding the composition to the soil is also provided.

## Description

### Technical Field

The present disclosure relates to a technology to facilitate symbiosis between plants and microbes. More specifically, the present disclosure relates to compositions and methods for promoting the colonization of arbuscular mycorrhizal fungi in plants and facilitating their symbiosis.

### Background Art

About 90% of the terrestrial plants establish symbiotic relationship with fungi in their roots, and this symbiosis helps the plants to efficiently utilize essential elements and other nutrients present in the soil. The symbiotic structure, formed by a fungus and a plant when such a fungus invades the plant root to become symbiotic, is called a "mycorrhiza", and the symbiotic phenomenon is called "mycorrhizal symbiosis".

Arbuscular mycorrhizal (AM) fungus (also called VA (Vesicular Arbuscular) mycorrhizal fungus) is a type of endomycorrhizal fungus whose hyphae invade the cell wall of the plant root to interact with the cytoplasm. It is believed that most agricultural plants benefit from symbiosis with arbuscular mycorrhizal fungi to improve the efficiency of utilization of phosphorus (which may be more specifically in the form of phosphate) and other nutrients present in the soil. In this symbiosis, regardless of differences in the types of plants and mycorrhizal fungi, a common structure called Arbuscule is formed. Arbuscule is a structure formed by a hypha that has invaded within the contour of plant root cell wall, become invaginated into plant plasma membrane (rather than penetrating it), and highly branched to become a tree-like shape. The area of interaction between the mycorrhizal fungus and the plant cytoplasm is significantly increased by this tree-like structure of the arbuscule. On the other hand, the hyphae of the mycorrhizal fungus, which extend and branch outside the plant cell wall, enable the procurement of nutrients from wider soil regions and increase the chance of infecting new plant root regions.

Phosphorus is an essential element for living organisms, and plants gain the ability to grow in relatively phosphorus-poor soils by mycorrhizal symbiosis. The global problem of depletion of phosphate rock sources could affect the production of phosphorus-based fertilizers. Soils in Japan also pose a problem of high phosphorus adsorption, which prevents plants from absorbing it. Therefore, it is expected that more active utilization of mycorrhizal fungi in agriculture will be beneficial.

However, while mycorrhizal fungi-containing soil amendments which use the arbuscular mycorrhizal fungi as active components (VA mycorrhiza fungal agents) are already commercially available, they are expensive, and the stability of their effect has not been necessarily satisfactory. Therefore, even though the VA mycorrhiza fungal agent is the only one microbial material officially recognized by the Soil Fertility Enhancement Act, in reality its use has not become so widespread.

Patent Document 1 discloses a method of promoting symbiosis wherein an inhibitor of gibberellin synthesis is administered at an amount equal to or lower than the amount suitable as a growth inhibitor, thereby promoting symbiosis of the plant with the mycorrhizal fungi. Patent Document 2 discloses an agent for promoting arbuscular mycorrhizal fungus colonization in plants, the agent comprising chitin oligosaccharides and/or chitin nanofibers. Patent Document 3 discloses an agent directed to arbuscular mycorrhizal fungi for promoting symbiosis, the agent comprising oxidized glutathione and/or cystathionine as an active component.

Non-Patent Document 1 discloses that the plant hormone gibberellin promoted the formation of Paris-type mycorrhizas by an arbuscular mycorrhizal fungus in lisianthus whereas the same hormone inhibited the formation of Arum-type mycorrhizas in chive etc. Non-Patent Document 2 discloses that a strigolactone, a plant component, promotes the hyphal branching of arbuscular mycorrhizal fungi.

The strigolactone is an especially promising agent, e.g. from the viewpoint of stability of the effect, for promoting arbuscular mycorrhizal fungus colonization. However, it also has undesired effects such as inhibiting the shoot branching of the plant itself and stimulating seed germination of parasitic plants such as Striga and Orobanche, which parasitize agriculturally important crops. It also has shortcomings such as being unstable in water and being very expensive.

### Citation List

### Patent Documents

Patent Document 1: JP 2017-38562 A
Patent Document 2: JP 2020-171216 A
Patent Document 3: JP 2021-159019 A

### Non-Patent Documents

Non-Patent Document 1: Plant Cell Physiol, 61(3): 565-575 (2020)
Non-Patent Document 2: Nature, 435, 824-827 (2005)

### Summary of the Invention

### Problem to Be Solved by the Invention

The environments for growing plants, for example, agricultural or horticultural plants, are diverse, and therefore providing new options for compositions and methods that can be used to promote symbiosis between plants and mycorrhizal fungi would be beneficial. Especially, the provision of compositions and methods that can act directly on the mycorrhizal fungi side, as opposed to compositions and methods which are thought to act mainly on the plant side (like those described in Patent Documents 1 and 2), may be preferable in view of the facts: (1) there is a need for improving the usefulness of the VA mycorrhiza fungal agents; and (2) the type of substances which stimulate the plants to release signal to the mycorrhizal fungi, such as gibberellin, may have highly varying plant responses depending on the type of the plants.

The embodiments of the present disclosure provide compositions and methods that can act on the arbuscular mycorrhizal fungi to enhance their ability to colonize plants and which can in turn promote their symbiosis with the plants.

### Solution to the Problem

During the course of studying physiological mechanisms on the plant side which may be involved in the induction of symbiosis with arbuscular mycorrhizal fungi, the present inventors discovered that gentiopicroside, a compound comprised in the roots of Gentianaceae plants, had an effect to promote hyphal branching of the arbuscular mycorrhizal fungi, and the compound when administered externally could promote arbuscular mycorrhiza fungal colonization to a variety of plants. Furthermore, the inventors discovered that other, structurally related compounds could provide similar effects.

The present disclosure includes the following embodiments.
[1] A composition for use in promoting colonization of an arbuscular mycorrhizal fungus, the composition comprising as an active component a secoiridoid glycoside, iridoid glycoside, or combination thereof.
[2] A composition for use in promoting colonization of an arbuscular mycorrhizal fungus, the composition comprising as an active component a glycoside in which a 3,4-dihydropyran ring skeleton forms at position 2 an O-glycosidic bond to a glucopyranose.
[3] The composition according to [1] or [2], wherein the active component is gentiopicroside, swertiamarin, loganin, geniposide, oleuropein, or any combination thereof.
[4] A composition for use in promoting colonization of an arbuscular mycorrhizal fungus, the composition comprising as an active component dried remains or an extract of a Gentianaceae plant.
[5] A kit for use in improving a soil, the kit comprising the composition of any one of [1] to [4] and an arbuscular mycorrhizal fungus.
[6] A method for promoting colonization of an arbuscular mycorrhizal fungus, the method comprising adding the composition of any one of [1] to [4] to the arbuscular mycorrhizal fungus.
[7] A method for enhancing an ability of an arbuscular mycorrhizal fungus present in a soil to colonize a root of a plant growing on the soil, by adding the composition of any one of [1] to [4] to the soil.
[8] The method of [7], further comprising adding an arbuscular mycorrhizal fungus to the soil.
[9] The method of [8] wherein the composition and the arbuscular mycorrhizal fungus are added to the soil in a form of a soil amendment comprising both.
[10] A method of producing a composition for use in promoting colonization of an arbuscular mycorrhizal fungus, the method comprising performing extraction from a Gentianaceae plant with water, organic solvent, or a mixture thereof to obtain an extract.

### Brief Descriptions of the Figures

[Figure 1] Figure 1 shows stereomicroscopic pictures showing hyphal branching states (top) and hyphal branching quantification graphs (bottom) obtained from treating the arbuscular mycorrhizal fungus, cultured on the gel media, with the test compounds. The test compounds were gentiopicroside (GPS) or swertiamarin (SWM).
[Figure 2] Figure 2 shows the results of similar experiments as in Figure 1, in which test compounds were loganin, geniposide, or oleuropein.
[Figure 3] Figure 3 shows the results of similar experiments as in Figures 1 and 2, in which a water-extract of Ryutan was used instead of the isolated test compounds.
[Figure 4] Figure 4 shows the results of experiments investigating the effects of the test compounds on the symbiosis of different types of plants with the arbuscular mycorrhizal fungus.
[Figure 5] Figure 5 represents the results of experiments showing that, unlike the strigolactone (GR24), gentiopicroside (GPS) lacks the adverse property of inducing seed germination of the parasitic weed, common broomrape.
[Figure 6] Figure 6 shows the results of hyphal branching tests on a different type of arbuscular mycorrhizal fungus than that in Figure 1.
[Figure 7] Figure 7 shows the results of experiments investigating the effects of the Ryutan water extract on symbiosis between the plant and the arbuscular mycorrhizal fungus.

### Detailed Description of the Invention

In some embodiments, the present disclosure provides a composition for use in promoting colonization of an arbuscular mycorrhizal fungus, the composition comprising as an active component a secoiridoid glycoside, iridoid glycoside, or combination thereof. Hereinafter, an arbuscular mycorrhizal fungus may be referred to as an AM fungus. The groups of compounds called secoiridoid glycosides and iridoid glycosides are known per se, and they are known to exist mainly in plants. An iridoid glycoside is characterized by having a 3,4-dihydropyran ring skeleton (described below) which is fused to a five-membered ring (at the [c] fusion side of the pyran) and glycosylated at position 2 of the 3,4-dihydropyran ring skeleton. Opening the five-membered ring and substituting it with other groups provides a secoiridoid glycoside.

In some embodiments, the present disclosure provides a composition for use in promoting colonization of an AM fungus, the composition comprising as an active component a glycoside in which a 3,4-dihydropyran ring skeleton forms at position 2 an O-glycosidic bond to a glucopyranose. This description of the glycoside is also compatible with the secoiridoid glycosides and iridoid glycosides mentioned above. In the present disclosure, the term "3,4-dihydropyran ring skeleton" means the ring skeleton of the compound 3,4-dihydropyran, i.e. a six-membered hetero ring formed by one oxygen atom and five carbon atoms and having one unsaturation (i.e. a double bond) at the carbon atom next to the oxygen atom. The structure of the compound 3,4-dihydropyran is shown below.

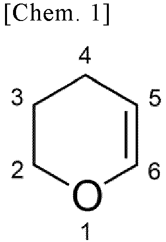

For the 3,4-dihydropyran ring skeleton of the present disclosure, positions of the atoms are referred to by the numbering shown in the structural formula above. The name of the compound "3,4-dihydropyran" originally signifies that the double bond between positions 3 and 4 of the parent compound pyran is saturated and additional hydrogen atoms are each bonded to positions 3 and 4, but the ring referred to by the term "3,4-dihydropyran ring skeleton" in the present disclosure does not need to have hydrogen atoms bonded to positions 3 and 4. For example, it may include an embodiment in which no hydrogen atom is bonded to position 3 or 4.

In fact, it has been found that the 3,4-dihydropyran ring skeletons comprised in the glycosides of the present embodiments can be modified by highly diverse substituents and still maintain the ability to promote colonization of the AM fungi. It therefore appears that the common 3,4-dihydropyran ring skeleton itself along with the glucopyranose ring described below contributes to the activity. Therefore, the term "glycoside in which a 3,4-dihydropyran ring skeleton forms at position 2 an O-glycosidic bond to a glucopyranose" in the present disclosure encompasses those having modifications with various substituents in addition to this basic structure.

For example, in some embodiments, the substituent at position 3 (R¹) of the 3,4-dihydropyran ring skeleton may be an alkyl group having 1 to 6 carbons or an unsaturated chain hydrocarbon group having 2 to 6 carbons, such as a vinyl or ethylidene group. In other embodiments, the substituent at position 3 (R¹) and the substituent at position 4 (R²) of the 3,4-dihydropyran ring skeleton may be linked to form a saturated or unsaturated five-membered ring together. That is, this five-membered ring is fused to the 3,4-dihydropyran ring skeleton. In some embodiments, position 5 (R³) of the 3,4-dihydropyran ring skeleton may have an ester group (-COO-R¹¹), wherein R¹¹ may be an alkyl group having 1 to 6 carbons, such as methyl, or an alkenyl group having 2 to 6 carbons. Alternatively, R¹¹ may be linked to the substituent at position 4 (R²) of the 3,4-dihydropyran ring skeleton to form a saturated or unsaturated six-membered hetero ring. That is, this six-membered hetero ring is fused to the 3,4-dihydropyran ring skeleton. In this particular embodiment, the 3,4-dihydropyran ring skeleton may further have a hydroxyl group at position 4. In specific embodiments, said alkyl group or unsaturated hydrocarbon group at position 3 (R¹) and said six-membered hetero ring fused at positions 4-5 may coexist. In other specific embodiments, said ester group at position 5 not forming a ring (R³) and said five-membered ring fused at positions 3-4 may coexist. In other specific embodiments, said alkyl group or unsaturated hydrocarbon group at position 3 (R¹) and said ester group at position 5 not forming a ring (R³) may coexist. In this particular case, the substituent at position 4 (R²) is typically a carboxymethyl group or an ester thereof (-CH₂-COOR¹²). R¹² may be for example an alkyl group having 1 to 6 carbons, or aryl alkyl wherein the divalent alkyl moiety may for example have 1 to 6 carbons (e.g., ethyl). In some embodiments, the hydrogen atom at position 6 of the 3,4-dihydropyran ring skeleton is not substituted (R⁴=H).

General formula (I) representing the glycoside in which a 3,4-dihydropyran ring skeleton forms at position 2 an O-glycosidic bond to a glucopyranose, described in the preceding paragraphs, is shown below.

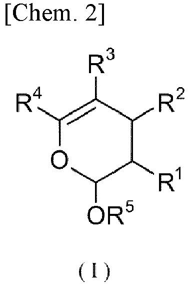

In the glycosides of these embodiments, position 2 of the 3,4-dihydropyran ring skeleton is bonded to an oxygen atom, and the glucopyranose (β-D-glucopyranose) (R⁵) forms a glycosidic bond via this oxygen atom. That is to say, a glucopyranose (or glucose) forms an O-glycosidic bond through its anomeric carbon (i.e., carbon at position 1 of the pyranose) to position 2 of the 3,4-dihydropyran ring skeleton.

Examples of glycosides that can be suitably used as an active component in the above-described embodiments include gentiopicroside, swertiamarin, loganin, geniposide, and oleuropein, and any combination thereof may be included as an active component as well. These glycosides are encompassed by general formula (I). Gentiopicroside is especially preferable in view of its high activity, and its extraction efficiency due to the high contents in the Gentianaceae plants. These compounds appear stable in water.

A structural formula of gentiopicroside is shown below.

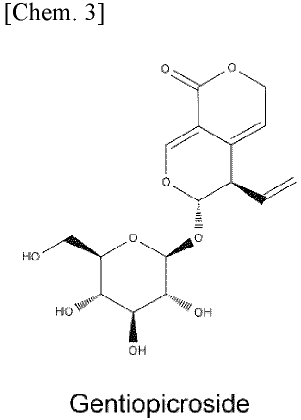

A structural formula of swertiamarin is shown below.

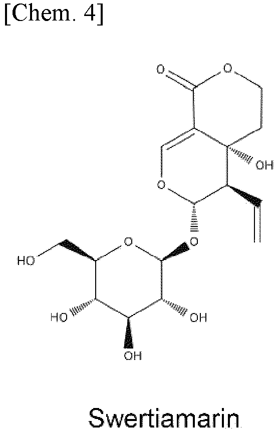

A structural formula of geniposide is shown below.

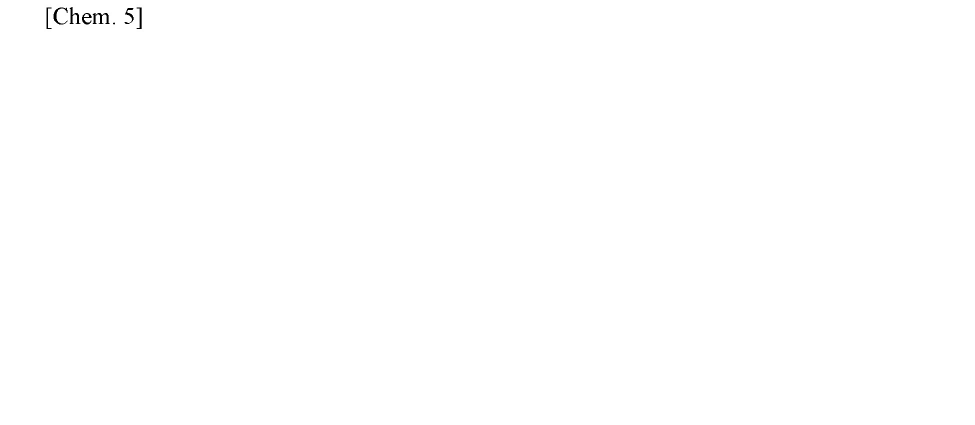

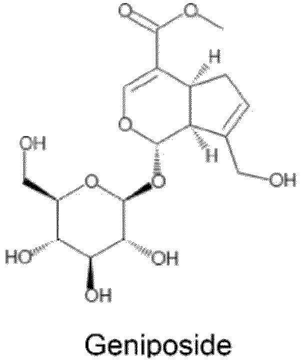

A structural formula of loganin is shown below.

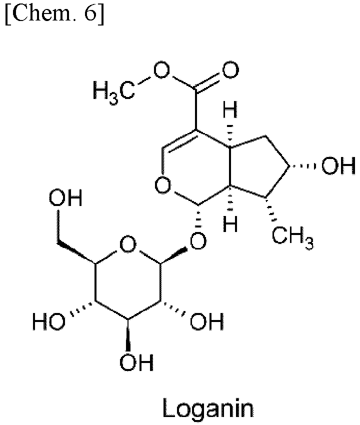

A structural formula of oleuropein is shown below.

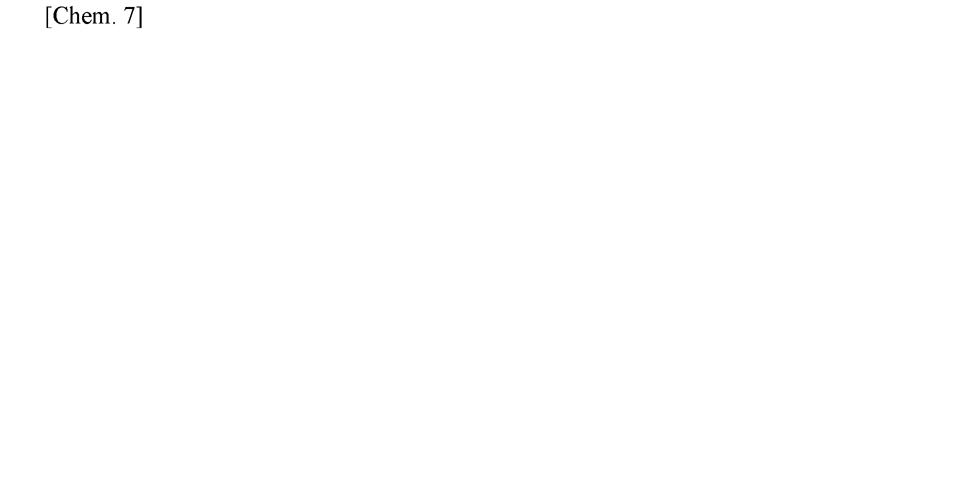

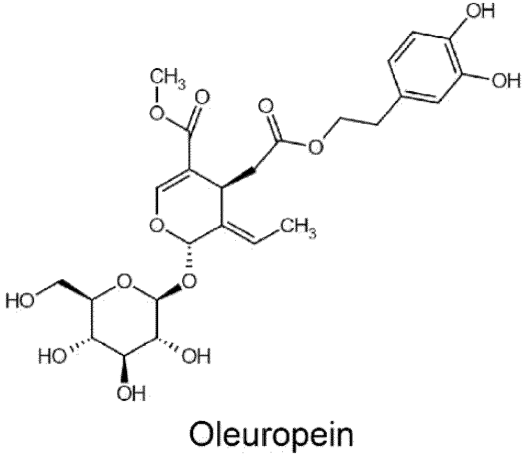

These are only non-limiting examples, and other various compounds which are synthesized or naturally sourced glycosides having the 3,4-dihydropyran ring skeleton may provide similar activity. The active compounds described above may be included in the composition as isolated compound(s). An "isolated compound" herein refers to a condition in which the compound is substantially separated from other organic compounds that were accompanying it in the source material such as a plant extract or a reaction solution for synthesis, such that the proportion of such accompanying organic compounds originating from the source material is less than 50% by mass (i.e., less than the amount of the compound of interest). Methods for synthesizing iridoids and secoiridoids (including glycosides thereof) are known (see, e.g., Buechi et al., "Total Synthesis of Loganin", J. Am. Chem. Soc. 1970, 92, 7, 2165-2167; Isoe, "Progress in the Synthesis of Iridoids and Related Natural Products", Studies in Natural Products Chemistry. 1995, 16: 289-320; Kouda at el., "Recent Advances in Iridoid Chemistry: Biosynthesis and Chemical Synthesis", Chem Asian J. 2020 Nov 16;15(22):3771-3783), and a person skilled in the art can modify substituents of the synthesized or naturally sourced compounds based on ordinary knowledge.

The active component in the present disclosure may be derived from a plant. For example, Ryutan, a crude drug produced by drying Rindou root and rhizome, is known to comprise secoiridoid glycosides such as gentiopicroside as main components. Semburi is also a Gentianaceae plant, its flowers, leaves, stems, and roots all have strong bitterness, and the infusion brewed from a dried whole plant of Semburi is drunk as intensely bitter "Semburi tea". Its bitterness components are also mainly secoiridoid glycosides such as swertiamarin. In fact, the present inventors have found that water- or organic solvent-extracts from dried Gentianaceae plants can provide the effect of promoting colonization of the mycorrhizal fungi similar to that of the isolated compounds. It is believed that the secoiridoid glycoside contents are mainly responsible for this effect, but contribution from iridoid glycosides is not excluded. In other words, the active component of the composition for use in promoting colonization of an arbuscular mycorrhizal fungus can be provided in the form of dried remains or an extract of a Gentianaceae plant.

Accordingly, in some embodiments, a composition for use in promoting colonization of an AM fungus is provided, the composition comprising as an active component dried remains or an extract of a Gentianaceae plant. Any of the root, stem, rhizome, leaf, and flower of the Gentianaceae plant may be used, as well as a combination thereof (for example, an underground or above-ground part of the plant, or a whole plant). The root is especially preferable. In these embodiments, the active compounds for promoting AM fungus colonization described above are not isolated, and they are believed to act in the mixture with other Gentianaceae plant-derived organic compounds. The extract may be a partially purified extract. Needless to say, these compounds may promote AM fungal colonization not only in the Gentianaceae plants but also in non-Gentianaceae plants.

Dried remains in the present disclosure are defined as a material having a water content of 15% by mass or lower. It is preferable, from the view point of dispersibility of the active compounds and uniform applicability to the soil, that the dried remains are finely fragmented, or pulverized, for example to the diameter (maximum diameter) of no larger than 5 cm, no larger than 3 cm, or no larger than 1 cm. Following the application to the soil, the active compounds will be gradually released from the dried remains into the soil. However, from the viewpoint of fast and efficient availability of the active compounds, an extract as described above may be more preferable. A water extract may be prepared by contacting a plant or fragment thereof with water having a temperature of e.g. 50°C or higher, 70°C or higher, or 90°C or higher, or boiling water, to extract water-soluble components. It is also known that instead of, or in addition to, water, an organic solvent may be used as an extractant to extract secoiridoid glycosides from Gentianaceae plants (see for example "Gentiana" in the Japanese Pharmacopoeia, 14th edition). Examples of suitable organic solvents include, but are not limited to, methanol, ethanol, and a combination thereof. An organic solvent showing miscibility with water is preferable. A person skilled in the art can identify a suitable extractant for extracting secoiridoid glycosides or the like from Gentianaceae plants based on ordinary knowledge in the art without undue experimentation. Time for extraction can, for example, be, but is not limited to, 5 minutes - 24 hours or 10 minutes - 1 hour. As described above, for extraction, an auxiliary solvent besides water may have been added. The amount (mass) of the auxiliary solvent in the extractant may be smaller than that of water. The plant or fragment thereof used as a source of extraction may be dry, and for example, the dried remains described above may be used. Drying and/or fragmenting of the plant is preferable as it breaks the plant tissues and facilitates release or extraction of the active compounds. The term water extract means a composition comprising components extracted by water as described above, and it may refer to the extract which has been dried or lyophilized, following the extraction, to remove water. Ditto for organic solvent extracts and the like. The present disclosure in one aspect provides a method of producing a composition for use in promoting colonization of an AM fungus, the method comprising performing extraction from a Gentianaceae plant with water, organic solvent, or mixture thereof to obtain an extract, as described herein. This extract, as is, can be used as an active component for the composition for use in promoting colonization of an AM fungus. Alternatively, this extract can be fractionated by a method known to those skilled in the art (for example, using the differences in molecular weights, binding to an affinity column, solubility to a solvent, etc.), to purify, or isolate, the secoiridoid glycoside and/or iridoid glycoside or the glycoside in which a 3,4-dihydropyran ring skeleton forms an O-glycosidic bond at position 2 to a glucopyranose.

Examples of the Gentianaceae plants that can be used in these embodiments include, but are not limited to, Gentiana scabra (Tou Rindou), Gentiana manshurica (Manshu Rindou), Gentiana triflora (Ezo Rindou), Gentiana lutea (Gentiana), Swertia japonica (Semburi), and any combination thereof.

The composition for use in promoting colonization of an AM fungus according to the present disclosure may be provided, and may be spread over or mixed into the soil, as a solution (for example an aqueous solution) or in a dried state (for example by being attached to or mixed with a particulate solid substrate as described below). Alternatively, the composition may be directly applied to a cultured AM fungus. The composition for use in promoting colonization of an AM fungus, according to the present disclosure, can enhance hyphal branching of the AM fungus and in turn facilitate colonization of the AM fungus in the plant root and symbiosis, which is a consequence of the colonization.

The present disclosure, in one aspect, provides a kit for use in improving a soil, the kit comprising any of the compositions described above and an AM fungus. The AM fungus may include that in the form of a spore. It should be appreciated that, by applying (e.g., spreading or mixing) said composition and the AM fungus to the soil, the ability of the AM fungus to colonize the plants growing on the soil may be enhanced, and thus a soil environment may be provided in which efficiency of utilization of phosphorus, sulfur, nitrogen, and other nutrients by the plants is improved, i.e., improvement of the soil may be achieved. The arbuscular mycorrhizal fungi are known to be eumycetes of phylum Glomeromycota, each of which employs the common mechanism called arbuscule for symbiosis and thus has a potential to be affected by the composition according to the present disclosure, but the most common examples are those of the order Glomerales, especially Glomeraceae family, especially Glomus and Rhizophagus genera, for example Rhizophagus irregularis (formerly known as Glomus intraradices). **In** some embodiments, the composition and the AM fungus in the kit are provided in separate packages. **In** another embodiment, the composition and the AM fungus in the kit are provided as a mixture contained in a same package. The kit may include instructions for improving a soil by using the composition and the AM fungus.

The mycorrhiza fungi contained in the commercially available VA mycorrhiza fungal agents are typically provided in a form attached to or mixed with particulate solid substrates such as vermiculite, perlite, diatomite, clay minerals, and silica-based minerals. Likewise, the composition for use in promoting colonization of an AM fungus in the kit of the present embodiment may be provided in a form attached to or mixed with particulate solid substrate, either together with the mycorrhizal fungus or in a package separate from the mycorrhizal fungus. The particulate solid substrate may for example have a diameter (maximum diameter) of no larger than 3 cm, no larger than 1 cm, or no larger than 0.5 cm. Alternatively, the composition may be included in a form of a solution, e.g. an aqueous solution. The embodiments like these are applicable when the composition is provided as part of a kit as well as when the composition is provided independently, and they are convenient for practical use of the composition for use in promoting colonization of an AM fungus in agricultural or horticultural settings.

**In** some embodiments, a method for promoting colonization of an AM fungus is provided, the method comprising adding any of the compositions for use in promoting colonization of an AM fungus described above (or an active component thereof) to the AM fungus. **In** these embodiments, the adding may be performed either in the presence or absence of the plant. For example, the compositions for use in promoting colonization of an AM fungus may be added to the AM fungus which is cultured on a gel culture medium or in soil, and as a result, the active component of the composition comes in contact with the AM fungus.

In some embodiments, provided is a method for enhancing an ability of an AM fungus present in a soil to colonize a root of a plant growing on the soil, by adding the composition for use in promoting colonization of an AM fungus (or an active component thereof) to the soil. This method can be understood as a method of improving the soil. At the time of adding, the plant may be already present in the soil, or the plant may be planted in the soil or the soil may be added to the plant after the adding. In these embodiments, the AM fungi originally present in the soil may be utilized, but additionally or alternatively, the method may further comprise adding an AM fungus to the soil. Embodiments in which the composition for use in promoting colonization of an AM fungus and the AM fungus are added to the soil at different time points from each other (that is, the composition is added before the AM fungus, or vice versa), and embodiments in which they are added to the soil simultaneously, are both contemplated. If the composition for use in promoting colonization of an AM fungus and the AM fungus are added to the soil simultaneously, they may be added to the soil in a form of a soil amendment comprising both. It should be appreciated that a kit, of the embodiment described above for example, may be utilized for simultaneously or sequentially applying the composition for use in promoting colonization of an AM fungus and the AM fungus to the soil as described above. Adding a composition comprising the active component for promoting colonization of the mycorrhizal fungus and/or comprising the mycorrhizal fungus to the soil may include, for example, spreading them over the soil, mixing them into the soil, etc.

### Examples

Below, examples are shown to describe embodiments of the present disclosures in greater details. However, these are examples for illustrative purpose, and the present invention is not limited to these examples. For example, the compounds than can provide active components for the compositions for use in promoting colonization of an AM fungus are not limited to those exemplified here.

### [Example 1: Activity of isolated secoiridoid glycosides and iridoid glycosides to promote hyphal branching]

### (1) Materials

Test fungi
- Arbuscular mycorrhizal fungus Rhizophagus irregularis DAOM197198 (Premier Tech Biotechnologies)

Test reagents
- Gastrografin (Bayer Yakuhin, Ltd.)
- rac-GR24 (StrigoLab)
- Gentiopicroside (abbreviated as GPS), Swertiamarin (abbreviated as SWM), Loganin, Geniposide, and Oleuropein (all >90% purity chemicals purchased from Tokyo Chemical Industry Co., Ltd.)

### (2) Separating and culturing spores of the AM fungi

The spores of the AM fungi were separated and cultured on gels according to the conventional procedures outlined below.
1. In a 50 mL centrifuge tube, Gastrografin aqueous solutions having different densities were gently layered in the order of 50% (10 mL), 32% (5 mL), 16% (5 mL), and 8% (5 mL), and finally 5 mL of a spore suspension of R. irregularis (hereinafter called the AM fungus) was loaded, and centrifugation was performed at 500 g for 10 minutes.
2. The spores separated in the density gradient from the above were collected on a cell strainer and washed with sterilized water 3 times.
3. The spores were suspended in sterilized water at 4000 spores/mL.
4. A 4 µL drop (about 8 spores) each of the spore suspension diluted to 2000 spores/mL was put in the center portion of the M medium (New Phytol. 108:211-218), which had been dispensed in a 24-well plate at 350 µL each.
5. After it was left for 5 minutes to allow excess moisture to evaporate, 200 µL each of 0.3% Phytagel (3 mM MgSO₄) at a temperature of about 50°C was gently poured over the spores. Three to four wells were prepared per treatment group.
6. They were cultured in the dark for 5 days at 25°C to let the spores germinate in advance.

### (3) Test treatments of the AM fungi and measurement of hyphal branching numbers

1. rac-GR24 (hereinafter denoted as GR24), a synthetic strigolactone known to have a hyphal branching activity for the AM fungi, was used as a positive control. From the GR24 stored in acetone, the solvent was removed *in vacuo,* and GR24 was resuspended in distilled water and sterilized with a 0.45 µm filter.
2. For the other test substances, dry solids were dissolved in distilled water and filter-sterilized as above.
3. 200 µL each of the test solutions was added dropwise onto the Phytagel media comprising the germinated spores obtained in (2) above. The AM fungi were treated using sterilized water as a negative (Mock) control and with GR24 at a final concentration of 100 nM as a positive control.
4. Culturing was performed in the dark for 7-10 days at 25°C.
5. Hyphal branching numbers were measured under a stereomicroscope. The hyphae that extended and branched directly from the hyphal stem had complex structures and branched well irrespective of the treatment conditions, and therefore they were excluded from the measurements of hyphal branching numbers.

### (4) Results

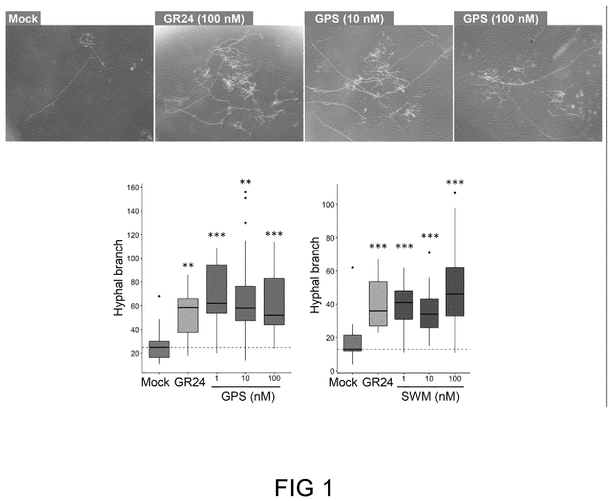

Figure 1, top panels show stereomicroscopic pictures of the branching hyphae, and bottom panels show the graphs for the hyphal branch numbers measured per spore. The number of hyphal branching is a parameter directly related to the efficiency of colonization to plant roots. A significant increase in the hyphal branch number was confirmed in the positive control treatment, 100 nM GR24, compared to the Mock treatment. Similarly, significantly high activities for promoting hyphal branching were shown for the secoiridoid glycosides Gentiopicroside (GPS) and Swertiamarin (SWM) over a wide concentration range tested, namely 1 to 100 nM. These results have revealed that GPS and SWM have the activity comparable to or greater than that of GR24 which is known to have an activity to promote hyphal branching of AM fungi. (n > 10, **: P < 0.01, ***: P < 0.001 in Steel test.)

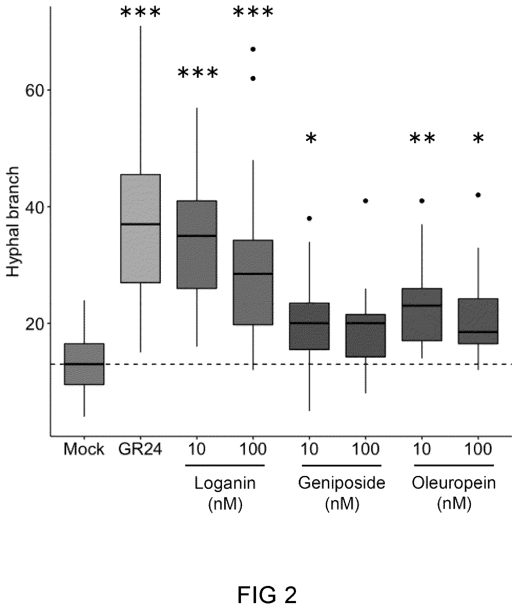

More surprisingly still, it was found that Oleuropein, which has the 3,4-dihydropyran ring skeleton and the glucopyranose ring moiety in common with these secoiridoid glycosides but has largely different modifications in other portions, as well as Loganin and Geniposide which are iridoid glycosides, also had the activity for the AM fungi to promote hyphal branching (Figure 2). The activity of Loganin was comparable to those of Gentiopicroside and Swertiamarin and the positive control GR24. Geniposide and Oleuropein appeared to have lower activities compared to GR24 and Loganin but still increased the hyphal branch numbers significantly compared to the negative control. (n > 10, *: P < 0.05, **: P < 0.01, ***: P < 0.001 in Steel test.)

### [Example 2: Activity of Gentianaceae plant water extract to promote hyphal branching]

In this example, Ryutan (Nakaya Hikojuro Yakkyoku) which is a crude drug produced by drying root and rhizome of Rindou (Gentiana spp.) was used. Extraction was performed by putting 10 g of Ryutan dry chips in 500 mL tap water and heating it by boiling for 10 minutes. Heating was stopped, and the cooled extract solution was filtered through a filter paper and a 0.45 µm filter. The concentrations of gentiopicroside and swertiamarin in this Ryutan extract solution were quantified by HPLC and found to be about 3 mM for gentiopicroside and about 300 µM for swertiamarin.

The extract solution, which had been filter-sterilized as above, was diluted 10⁴ - 10⁶ folds (corresponding to 3 - 300 nM GPS and 0.3 - 30 nM SWM), and used for treating the AM hyphae prepared and cultured as in Example 1. Hyphal branch numbers were measured 7 days later.

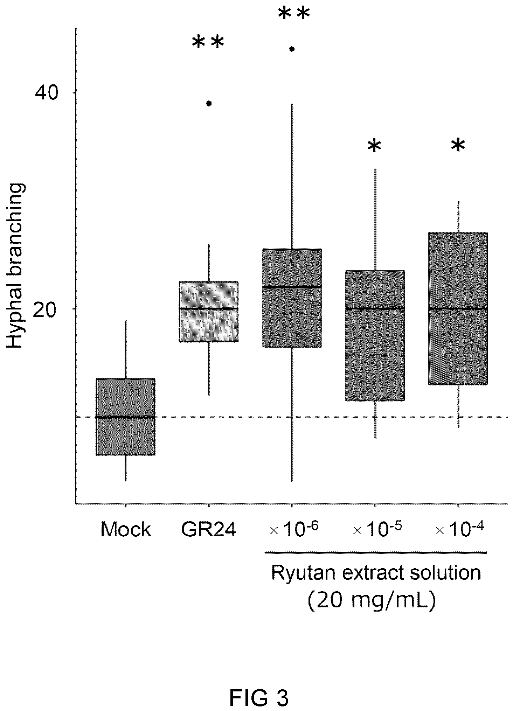

As a result, the water extract of Ryutan showed an activity to promote hyphal branching comparable to that of GR24, as shown in Figure 3. (n > 8, *: P < 0.05, **: P < 0.01 in Steel test.)

### [Example 3: Increase of symbiosis with plants - isolated secoiridoid glycosides]

To confirm that the identified active compounds not only promote hyphal branching of the AM fungi but also actually lead to increases of colonization and symbiosis with diverse plants, tests were carried out using lisianthus (Eustoma grandiflorum cv. Pink Thumb), tomato (Solanum lycopersicum L. cv. Sugar Lump), and chive (Allium schoenoprasum) as host plants.

Culturing of the host plants and AM fungal colonization and treatments with the active compounds were performed according to the following procedures.
(1) Plant box was assembled in 3 tiers: the top tier served as a lid having openings for ventilation, the middle tier was a box to which the culture soil (300 mL river sand) had been added, and the bottom tier was an empty box.
(2) The plant box of (1) above was sterilized by autoclaving (121°C, 20 minutes).
(3) To the culture soil of the middle box, 1000 spores of the AM fungi and 50 mL of the Hoagland hydroponic solution at a 1/10 concentration were added, and stirred with forceps. The active compounds such as GPS were added to the Hoagland hydroponic solution to treat the AM fungi and host plants.
(4) In order to prevent drying of the culture soil, 50 mL of the Hoagland hydroponic solution at a 1/10 concentration was also added to the bottom box. A twisted piece of KimWipe was placed through a hole provided at the bottom of the middle box, with the lower tip of the KimWipe soaked into the solution in the bottom box, and therefore the culture soil was maintained without becoming dry.
(5) Six seedlings of each host plant were planted per plant box, and cultured for 4 weeks in a culture chamber set at 24°C and 14 hour light cycle.
(6) After completing the culturing period above, the root of each host plant was sampled and preserved in an FAA fixative solution.

Colonization rates of the AM fungi were measured according to the following procedures.
(1) The root fixed in said fixative solution was cut into 1 cm fragments and immersed in a 10% KOH aqueous solution, and heated at 90°C for 15 minutes.
(2) After neutralizing the root in a 2% HCl aqueous solution, it was treated for staining with 0.05% trypan blue (1% trypan blue aqueous solution diluted 20 times with lactic acid) at 90°C for 15 minutes.
(3) The stained root fragments, 10 each, were aligned on a slide glass such that they were parallel to the long sides of the glass, and covered with a cover slip.
(4) Using a light microscope having an ocular lens fitted with an ocular micrometer, the stage was moved perpendicular to the root (in the Y-axis direction), and when the crosshair of the ocular micrometer touched the root, presence or absence of AM fungal hyphae in the root within the field of view was examined. The rate of hyphal invasion, i.e. colonization rate, as a total was calculated according to the following formula.
Rate of hyphal invasion (%) = (number of fields of view in which hyphae were seen / number of fields of view observed) × 100

The rate of arbuscule formation was calculated according to the above formula by replacing the numerator in parentheses, on the right-hand side of the equation, with "number of fields of view in which arbuscules were seen".

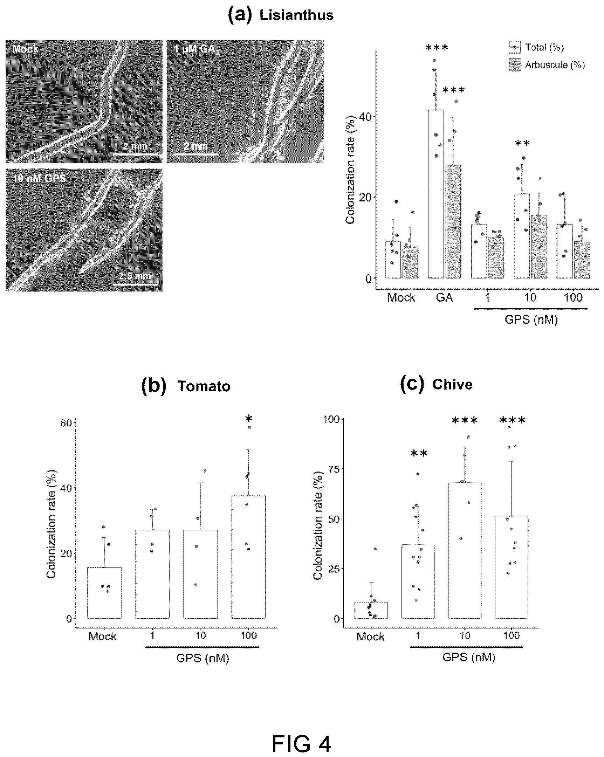

As shown in the results of Figure 4a (right), when the lisianthus-AM fungus combination was treated with GPS, an increasing trend in root colonization events was observed compared to the mock treatment, and a statistically significant increase was confirmed at the concentration of 10 nM. With the GPS treatments under this particular experimental condition, although the AM fungal colonization rates were overall lower than with the positive control 1 M gibberellin (GA), outside-of-root hyphae of the AM fungi that were vigorously branching in the rhizosphere were observed (Figure 4a, left). (n = 6, **: P < 0.01, ***: P < 0.001 in Dunnett's test.)

Figures 4b and 4c show the results for tomato and chive, respectively. Maximum average colonization rates of the AM fungi were obtained at the GPS concentrations of 100 nM for tomato and 10 nM for chive. For chive, a statistically significant increase of AM fungal colonization rate was confirmed even at 1 nM GPS which was the lowest concentration tested. (n = 4-12, *: P < 0.05, **: P < 0.01, ***: P < 0.001 in Dunnett's test.)

### [Example 4: Bioassay using common broomrape seeds]

The seeds of the genus Orobanche and genus Striga plants, which are root-parasitic weeds, have the property to germinate in response to strigolactones released from the roots of agricultural plants which serve as hosts. These root-parasitic weeds are causing serious damage to crop production worldwide. This poses a hindrance to the external application of strigolactone as an agent for promoting arbuscular mycorrhizal fungus colonization. In the present Example, it was tested whether the secoiridoid glycoside, which exhibited a high activity to promote hyphal branching of the AM fungi, would also have an activity of inducing germination of the seeds of the root-parasitic weeds.

Effect of gentiopicroside (GPS) on the germination of common broomrape (Orobanche minor), a parasitic weed of the genus Orobanche, was investigated. Experiments were performed according to the following experimental procedures.
(1) Common broomrape seeds were placed in a nylon mesh that had been shaped into a pouch (2 cm × 2 cm × 2 cm) by a sealer, and the opening of the pouch was closed.
(2) The seeds, together with the pouch, were placed in a 1% sodium hypochlorite aqueous solution to sterilize the surface for 20 minutes, and then washed 3 times with sterilized water.
(3) The pouch containing the seeds was placed on a filter paper dampened with sterilized water, and allowed to absorb water for 10 days at 25°C in the dark.
(4) 6 mm paper discs contained in a 96-well plate were moistened by adding 15 µL distilled water dropwise.
(5) A new paper disc was placed on the paper disc of (4) above, and 20 µL of the test solution was added dropwise. Distilled water was used as a negative control (Mock) and 1 µM strigolactone (GR24) as a positive control. For the solvent for GPS, methanol was chosen due to its volatility.
(6) For treating the common broomrape seeds with both GR24 and GPS, one reagent was first added dropwise, and after complete evaporation of the solvent, the other reagent was then added.
(7) After the discs were left for 5 minutes to remove the solvent by evaporation, the common broomrape seeds of (3) above, which had been transferred to distilled water, were added together with the water as 5 µL drops.
(8) After 5 days of culturing at 25°C in the dark, the germination rates of the common broomrape were measured.

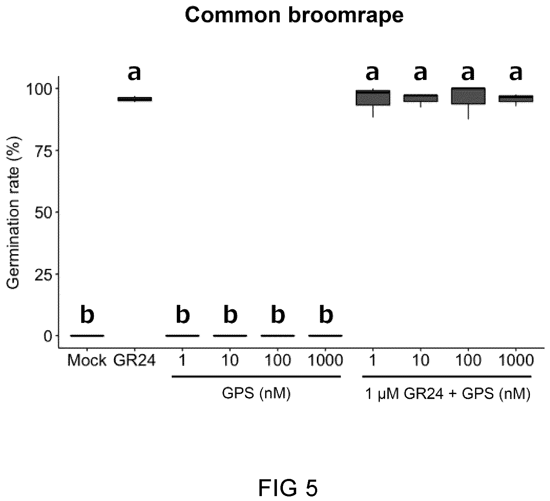

Results are shown in Figure 5. The vertical axis of the graph in Figure 5 shows the germination rate of the common broomrape seeds under this experimental condition. When the common broomrape seeds were treated with GR24, which is a synthetic strigolactone, nearly 100% of the seeds germinated. In contrast, the common broomrape seeds treated with 1 to 1000 nM GPS did not germinate at all. When a GPS treatment was performed in the concurrent presence of a GR24 treatment, the germination rates of the common broomrape seeds increased to nearly 100%. This indicates that it is not that GPS acts to suppress seed germination (if it had a general suppressive effect on seed germination, it could become unsuitable as a soil improver agent), but GPS simply lacks the undesirable effect of inducing germination of parasitic weed, which is inherent in strigolactones. As demonstrated in the preceding Examples, the GPS concentrations tested here against common broomrape were effective in promoting hyphal branching of the AM fungi and their colonization in host plants, and it is therefore appreciated that GPS can be used as an externally administered agent for promoting AM fungal symbiosis without the shortcomings of strigolactones, namely induction of seed germination of root parasitic weeds. (n = 3, P < 0.001 in Tukey test.)

### [Example 5: Activity of isolated secoiridoid glycosides to promote hyphal branching]

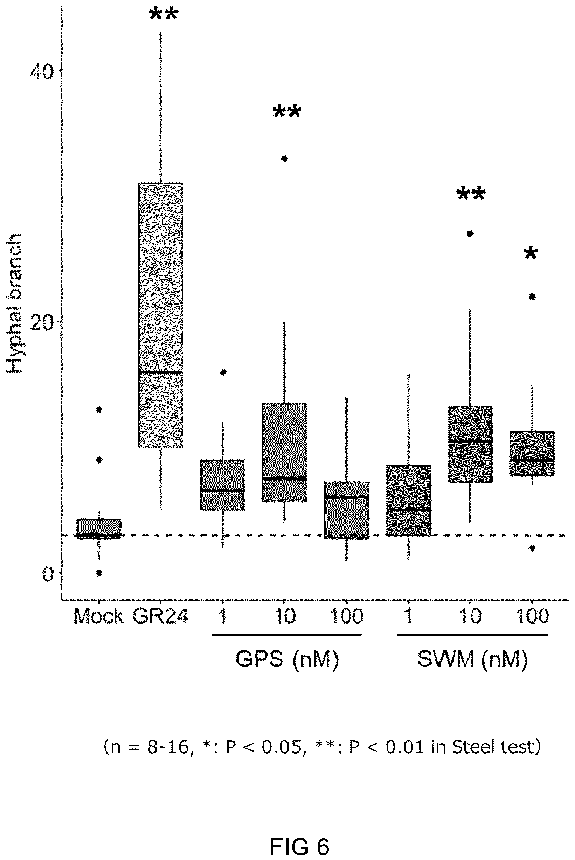

Essentially the same experiments as described in Example 1 were carried out, except that a different arbuscular mycorrhizal fungus, Rhizophagus clarus HR1, was used. As shown in Figure 6, significant increases of hyphal branching were confirmed for the treatments with gentiopicroside (GPS) and swertiamarin (SWM), as well as for the treatment with the positive control, 100 nM strigolactone (GR24). The compositions of the present disclosure can increase hyphal branching of diverse arbuscular mycorrhizal fungi included in the order Glomerales besides those exemplified here.

### [Example 6: Increase of symbiosis with plants - water extract of Gentianaceae plant]

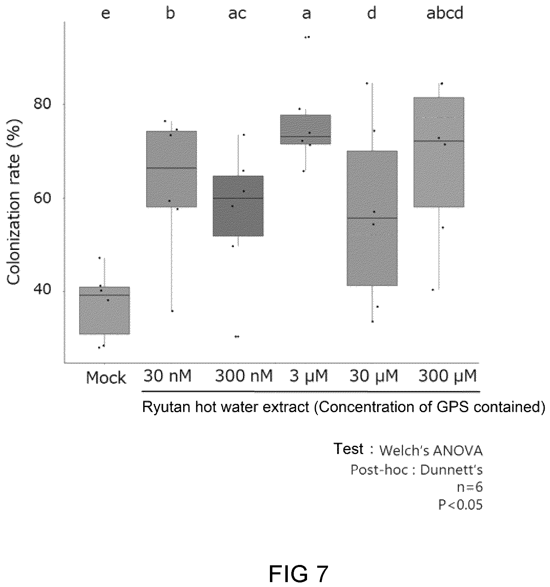

Essentially the same experiments as in Example 3 were carried out, except that the water extract of the Gentianaceae plant described in Example 2 was used instead of the isolated compounds. The subject plant tested for AM fungal colonization was chive. As shown in Figure 7, by administering the water extract of the Gentianaceae plant to the soil, statistically significant increases in the AM fungal colonization rates were confirmed. These results are similar to the experimental results of Figure 4c, which used the isolated compounds.

## Claims

1. A composition for use in promoting colonization of an arbuscular mycorrhizal fungus, the composition comprising as an active component a secoiridoid glycoside, iridoid glycoside, or combination thereof.

2. A composition for use in promoting colonization of an arbuscular mycorrhizal fungus, the composition comprising as an active component a glycoside in which a 3,4-dihydropyran ring skeleton forms at position 2 an O-glycosidic bond to a glucopyranose.

3. The composition according to claim 1 or 2, wherein the active component is gentiopicroside, swertiamarin, loganin, geniposide, oleuropein, or any combination thereof.

4. A composition for use in promoting colonization of an arbuscular mycorrhizal fungus, the composition comprising as an active component dried remains or an extract of a Gentianaceae plant.

5. A kit for use in improving a soil, the kit comprising the composition of any one of claims 1 to 4 and an arbuscular mycorrhizal fungus.

6. A method for promoting colonization of an arbuscular mycorrhizal fungus, the method comprising adding the composition of any one of claims 1 to 4 to the arbuscular mycorrhizal fungus.

7. A method for enhancing an ability of an arbuscular mycorrhizal fungus present in a soil to colonize a root of a plant growing on the soil, by adding the composition of any one of claims 1 to 4 to the soil.

8. The method of claim 7, further comprising adding an arbuscular mycorrhizal fungus to the soil.

9. The method of claim 8 wherein the composition and the arbuscular mycorrhizal fungus are added to the soil in a form of a soil amendment comprising both.

10. A method of producing a composition for use in promoting colonization of an arbuscular mycorrhizal fungus, the method comprising performing extraction from a Gentianaceae plant with water, organic solvent, or a mixture thereof to obtain an extract.
